# EUROPEAN PATENT APPLICATION

(11) **EP 3 318 196 A1**
(43) Date of publication of application: **09.05.2018**
(21) Application number: 16197839.0
(22) Date of filing: 08.11.2016
(51) Int. Cl.: A61B 17/00

(54) **A LIQUID DISPENSER**

(71) Applicant: Connexicon Medical Limited, 24 Dublin (IE)
(72) Inventor: Ledwidge, Éadaoin D., County Dublin (IE); Brennan, Martin O., Dublin, 18 (IE); Lennon, Oisín D., Dublin, 7 (IE); McHugh, Niall F., County Wicklow (IE)
(74) Representative: FRKelly

(57) **Abstract**

The present invention a liquid dispenser comprising a housing defining a chamber for releasably receiving an ampoule of a liquid to be dispensed, the body further defining an outlet to enable the liquid to be dispensed from the chamber; an actuator displaceable relative to the housing; wherein a section of the housing is adapted to permit a least a section of the actuator to be displaced into the chamber to apply a force, in use, to the ampoule.

## Description

### Field of the invention

The present invention is concerned with a liquid dispenser, in particular for use as a medical device that can express a liquid in a controlled manner, such as a cyanoacrylate based adhesive, for example for topical application to close wounds in a surgical setting.

### Background of the invention

Wound closure in surgical applications is conventionally achieved using sutures, staples, or similar mechanical closures, although these systems do suffer from a number of drawbacks. Thus use of sutures or staples can be both painful during the surgical procedure, and will often result in scaring such as puckering where the tissue is pierced by the closure and where the edges of the wound are drawn tightly together.

As an alternative to the above mechanical type closure systems it is also known to use a liquid adhesive to effect closure of a wound, for example a cyanoacrylate based adhesive. Such adhesives are conventionally dispensed from a suitable device directly onto the wound to be closed, the surgeon drawing the opposed edges of the wound into apposition before applying the adhesive along the wound, the adhesive being designed to cure on contact with the skin. The adhesive may require the action of a catalyst such as an initiator in order to effect curing of the adhesive, for example through the action of polymerization. Alternative adhesives may however not require the use of such a catalyst.

The adhesive is conventionally supplied in a closed container such as a glass ampoule, which is then broken in order to allow the adhesive to be dispensed by a suitable dispensing device into which the ampoule may be located. Breaking of the glass ampoule can however give rise to problems, in particular the safe management of the broken glass.

It is an object of the present invention to provide an improved liquid dispensing device which is capable of effectively dispensing liquid from such an ampoule while also safely managing the broken glass resulting from breaching the ampoule to access the liquid.

### Summary of the invention

According to the present invention there is provided a liquid dispenser comprising a housing defining a chamber for receiving an ampoule of a liquid to be dispensed, the body further defining an outlet to enable the liquid to be dispensed from the chamber; an actuator displaceable relative to the housing; wherein a section of the housing is adapted to permit a least a section of the actuator to be displaced into the chamber to apply a force, in use, to the ampoule.

Preferably, the section of the housing permitting access to the chamber is defined by a window in the housing.

Preferably, the actuator comprises an abutment shaped and dimension to pass through the window.

Preferably, the actuator comprises a cantilever arm projecting from the housing.

Preferably, the abutment is located at or adjacent to a free end of the cantilever arm.

Preferably, the actuator is reversibly displaceable relative to the housing.

Preferably, the actuator is hingedly mounted to the housing.

Preferably, the actuator is biased away from the housing.

Preferably, at least a portion of the actuator is formed from a resiliently deformable material.

Preferably, the outlet comprises a nozzle in fluid communication with the chamber.

Preferably, the nozzle is releasably retained on the housing in order to permit access to the chamber.

Preferably, the housing comprises a substantially cylindrical hollow body.

Preferably, the housing is rigid.

Preferably, the liquid dispenser comprises an ampoule of a liquid shaped and dimensioned to be received in the chamber.

Preferably, the ampoule comprises a breachable wall.

Preferably, the breachable wall is at least partially comprised of glass or brittle plastic.

Preferably, the liquid dispenser comprises an outer sleeve locatable to surround the ampoule and having an outlet for the liquid.

Preferably, the outer sleeve is deformable.

Preferably, the liquid dispenser comprises a filter disposed between the chamber and the outlet.

Preferably, the liquid dispenser comprises an initiator operable, on contact with the liquid, to alter the physical and/or chemical characteristics of the liquid.

Preferably, the initiator is located on an inner wall of the outer sleeve.

Preferably, the housing is at least partially transparent in order to provide visibility of the contents of the chamber.

Preferably, the housing may be shaped and dimensioned to provide a desired volume to the chamber.

Preferably, the liquid dispenser comprises a cartridge comprising the ampoule and the outer sleeve and optionally the filter

### Brief description of the drawings

The present invention will now be described with reference to the accompanying drawings, in which:
Figure 1 illustrates a perspective view of a liquid dispenser according to a preferred embodiment of the present invention;
Figure 2 illustrates an alternative perspective view of the liquid dispenser;

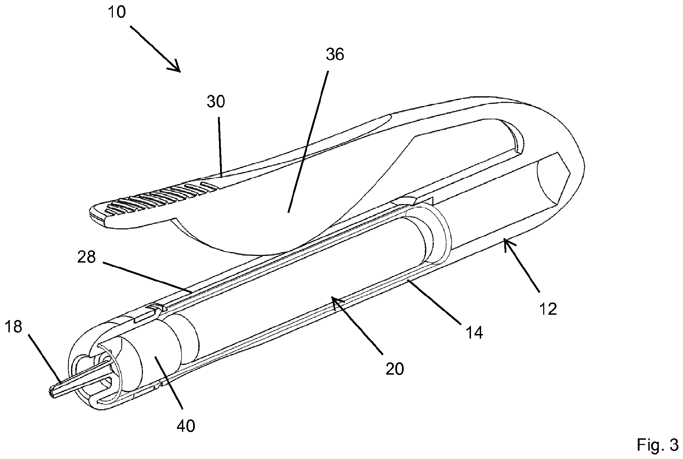

Figure 3 illustrates a sectioned view of the liquid dispenser with an ampoule of a liquid contained therein;

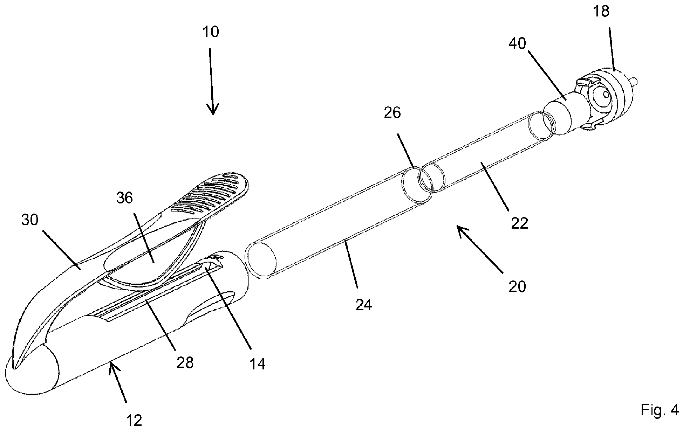

Figure 4 illustrates an exploded perspective view of the liquid dispenser;
Figure 5 illustrates a side elevation of the liquid dispenser with an actuator in a disengaged position;
Figure 6 illustrated a sectioned elevation of the liquid dispenser as illustrated in Figure 5;
Figure 7 illustrates a side elevation of the liquid dispenser with the actuator in a partially engaged or depressed position;
Figure 8 illustrated a sectioned elevation of the liquid dispenser as illustrated in Figure 7;
Figure 9 illustrates a side elevation of the liquid dispenser with the actuator in a fully engaged or depressed position;
Figure 10 illustrated a sectioned elevation of the liquid dispenser as illustrated in Figure 9;
Figure 11 illustrates a side elation of the liquid dispenser with the actuator fully depressed in order to dispense liquid;
Figure 12 illustrates a sectioned elevation of the liquid dispenser of Figure 11 with liquid being dispensed therefrom;
Figure 13 illustrates a perspective view of the liquid dispenser with a cartridge of liquid removed and shown alongside.

### Detailed description of the drawings

Referring now to the accompanying drawings there is illustrated a liquid dispenser, generally indicated as 10, having particular but not exclusive application in the dispensing of a liquid adhesive such as a cyanoacrylate based adhesive for topical application in the closure of a wound. The liquid dispenser 10 may however be used for dispensing other liquids, gels or flowable materials.

The liquid dispenser 10 comprises a body 12 which in the embodiment illustrated is of hollow cylindrical form, such as to define a chamber 14 on an interior thereof. It will however be appreciated from the following description of the configuration and operation of the dispensing device 10 that the body 12 may take any other suitable shape, and may vary in dimension in order to define varying volumetric capacities of the chamber 14 as will become apparent from the following description.

The body 12 additionally defines an outlet 16 at one end thereof which permits fluid to be dispensed, in use, from the chamber 14 onto a wound or other application site. The opposed end of the body 12 is closed. In the embodiment illustrated the mouth 16 is fitted with a nozzle in the form of a dispensing tip 18 which permits the controlled dispensing of liquid from within the chamber 14. It is envisaged that the dispensing tip 18 may be replaced or augmented with alternative terminations such as a brush head, sponge, foam or filter type applicator or the like. The dispensing tip 18 is releasably retained on the body 12 by any suitable means, for example one or more resiliently deformable tabs or barbs.

The liquid dispenser 10 is designed to receive and retain, preferably releasably, in the chamber 14, a single use container in the form of a cartridge 20, holding a discrete quantity of a liquid L to be dispensed, and as visible in the various sectioned views of the drawings. The body 12 and chamber 14 are thus preferably shaped and dimensioned to provide a complimentary fit such that the cartridge 20 is substantially immobilized within the chamber 14. A number of longitudinally extending ribs 38 may be provided on the inner wall of the chamber 14 in order to further improve the fit between the cartridge 20 and chamber 14.

The cartridge 20 comprises a cylindrical ampoule 22 within which the discrete quantity of liquid L is contained, the ampoule 22 being at least partially formed from a brittle material that may be ruptured or breached through the application of force, for example glass, rigid plastic or similar material. The ampoule 22 is also preferably translucent in order to allow a user to ascertain the presence of the liquid L therein. The cartridge 20 further comprises an outer sleeve 24 closely surrounding and enveloping the ampoule 22, the outer sleeve 24 being formed from a flexible or otherwise resiliently deformable material, for example a flexible polymer based material or the like. Again the outer sleeve 24 is preferably translucent in order to enable a user to ascertain the presence of the liquid L within the ampoule 22. While the ampoule 22 is completely closed to the external environment, the outer sleeve 24 includes at least one open end 26. In this way, and as will be described hereinafter in detail, the ampoule 22 may be broken or otherwise ruptured in order to enable the liquid L to flow outwardly therefrom, and the liquid L can then be dispensed from the open end 26 of the outer sleeve 24. The flexible outer sleeve 24 allows a deforming force to be applied through the outer sleeve 24 to the rigid ampoule 22 which will break once a suitable force is applied thereto. As the outer sleeve 24 is flexible it will not rupture or otherwise break and will thus retain a liquid impermeable barrier surrounding the broken ampoule 22 which will thus ensure that the liquid L is only capable of flowing outwardly through the open end 26. Although less preferred, it is also envisaged that the outer sleeve 24 could be omitted from the cartridge 20, and instead the outer sleeve 24 could be permanently or releasably located as an insert or lining within the chamber 14, into which the bare ampoule 22 could then be inserted.

Returning to the dispensing device 10, the body 12 further defines a port or window 28 which provides access to the chamber 14 and in the embodiment illustrated is in the form of an elongate slot running longitudinally to the body 12. The dispensing device 10 further comprises an actuator in the form of a cantilever arm projecting from the body 12, the cantilever arm 30 having a fixed end 32 secured to or formed integrally with the body 12, and an opposed free end 34, the cantilever arm 30 extending substantially longitudinally of the body 12 and at least partially overlying the window 28. The cantilever arm 30 is preferably formed integrally with the body 12, for example as a single molded, printed or otherwise formed component, and in the embodiment illustrated are designed to take the form of a conventional pen which is thus familiar to the user and is preferably dimensioned to be comfortable and practical for manual manipulation.

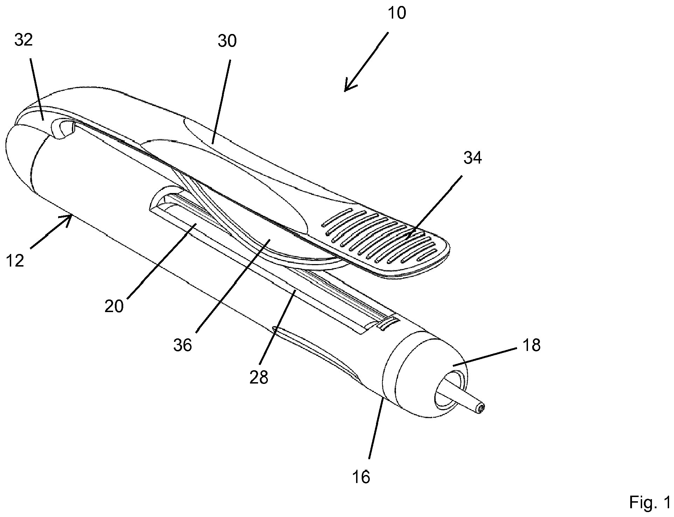

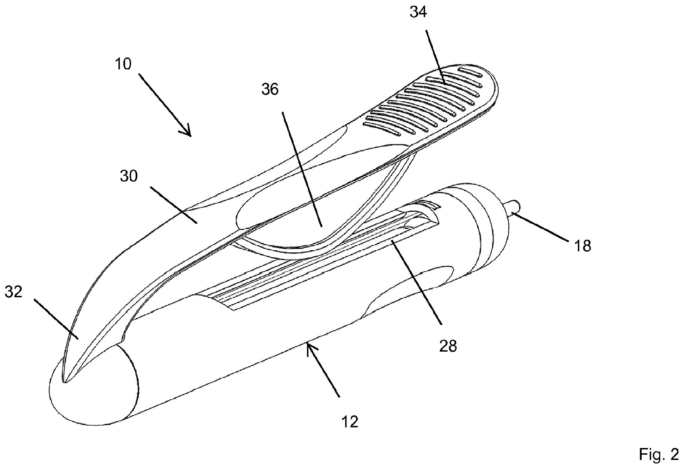

The cantilever arm 30 is displaceable relative to the body 12 through the application of manual force, most effectively about the free end 34 and in a direction radially inwardly of the body 12, this displacement preferably being facilitated by the use of a resiliently deformable material forming at least a part of the cantilever arm 30, although any other suitable means may be utilized in order to facilitate this displacement. In the preferred embodiment illustrated the cantilever arm 30 is reversibly displaceable relative to the housing, the fixed end 32 defining a live hinge between the cantilever arm 30 and the body 12. Thus when the manual force is released the cantilever arm 30 will return to the un-deformed position, for example as illustrated in Figures 1 and 2.

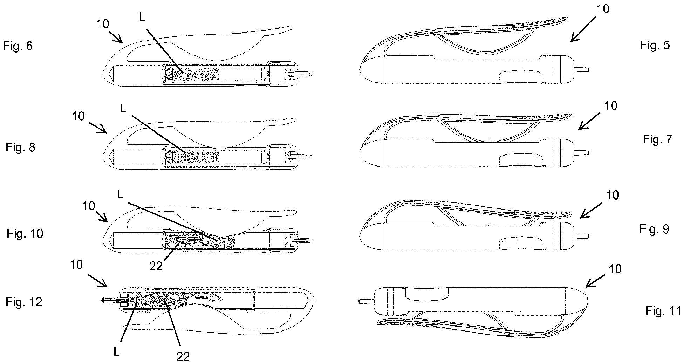

The cantilever arm 30 comprises an abutment 36 extending outwardly from the cantilever arm 30 towards the body 12, and preferably overlying a substantial portion of the window 28. The abutment 36 is dimensioned to sit outwardly of the body 12 when the cantilever arm 30 is in the un-deformed state, for example as illustrated in Figures 1 and 2, where no external force is being applied to the cantilever arm 30. The abutment 36 is however dimensioned, in particular in width, to be capable of passing through the window 28 into the chamber 14 when the cantilever arm 30 is deformed inwardly towards the body 12. Figures 7 and 8 illustrate the cantilever arm 30 in a partially depressed state showing the abutment 36 beginning to pass through the window 28. At this point it will be appreciated that the abutment 36 will contact the outer sleeve 24 of the cartridge 20, and the further application of force by an operator will begin to apply a force to the cartridge 20 thereby effectively clamping the cartridge 20 between the abutment 36 and the inner wall of the chamber 14 opposite the window 28. By applying for the force to the cantilever armed 30 the abutment 36 will rupture the ampoule 22, and in the case of a glass ampoule 22 will shatter the glass, thereby permitting the liquid L to flow from the ampoule 22 into the outer sleeve 24, as illustrated in Figures 5 and 6. The abutment 36 preferably comprises a curved profile such that the contact patch with the cartridge 20 is relatively small and thus the force transmitted to that point on the ampoule 22 will be magnified. In this way the actuator 30 will not be required to be displaced with significant force in order to break the ampoule 22, thereby ensuring ease of use of the liquid dispenser 10. In addition, the elongate form of the window 28 and the abutment 36 will ensure that lateral deflection of the actuator 30 does not occur during the application of force to the ampoule 22.

In use the cartridge 20 is oriented within the dispensing device 10 such that the open end 26 of the outer sleeve 24 is in register with the outlet 16 as defined by the dispensing tip 18. Thus by rupturing the ampoule 22 the liquid L can flow through the outer sleeve 24, via the open end 26 to the dispensing tip 18 to be dispensed from the dispensing device 10 as required. By repeatedly releasing and depressing the cantilever arm 30 a user can then effectively pump the liquid L from the chamber 14 as required.

In order to prevent any parts of the broken ampoule 22 from being dispensed along with the liquid L the liquid dispenser 10 preferably comprises a filter 40 located between the chamber 14 and the outlet 16, and in the embodiment illustrated is retained within the dispensing tip 18. The filter 40 is designed to permit the flow of the liquid L to exit from the dispensing tip 18, but to trap any pieces or particles of broken glass or the like from the ampoule 22.

Depending on the liquid L to be dispensed, it may be necessary to provide an initiator or similar catalyst which is adapted to effect a physical and/or chemical change in the liquid L such as to have the requisite properties for its intended application, for example to cure when used as a surgical adhesive. In the embodiment illustrated the inner wall of the outer sleeve 24 may be coated with a suitable initiator (not shown). In this way the initiator is maintained out of contact with the liquid L until the ampoule 22 is broken, at which point the liquid pouring from the ampoule 22 will come in contact with the initiator. In the case of a cyanoacrylate adhesive the initiator is operable to effect the polymerization of the monomer to a polymer. It will of course be appreciated that any other suitable initiator/catalyst may be employed if necessary.

The use of the cantilever arm 30 in order to effect the rupturing of the ampoule 22 and the subsequent pumping of the liquid L from the dispensing device 10 enables, through the use of leverage, significant force to be applied by a user in order to break the ampoule 22, and then allows substantial modulation of the pumping of the liquid L from the dispensing device 10, thereby allowing a greater degree of control in metering the flow of liquid L. In addition the use of the cantilever arm 30 to displace the abutment 36 through the window 28 enables the body 12 to be formed from a rigid material whilst still allowing controlled access to the cartridge 20 contained within the chamber 14, thereby essentially eliminating the possibility of a shard of broken glass from the ampoule 22 piercing the body 12 and potentially causing harm to the operator, were such a piece of glass to pierce the outer sleeve 24.

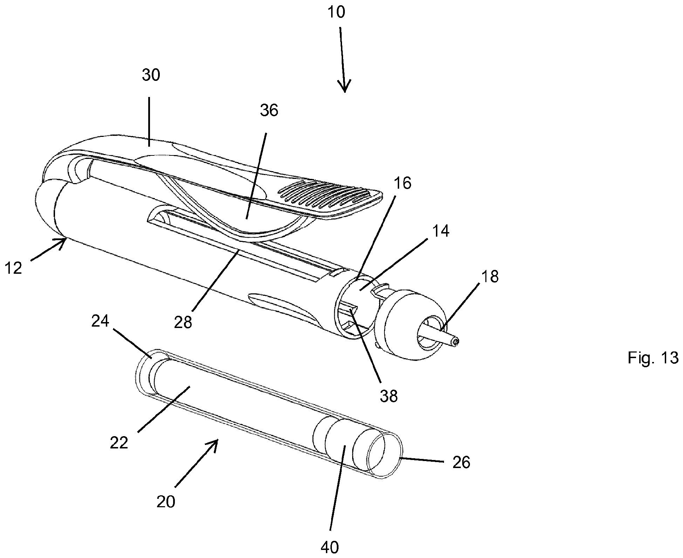

The dispensing device 10 may be a reusable device which allows an expired cartridge 20 or ampoule 22 to be removed from the chamber 14 and replaced. The dispensing tip 18 is therefore preferably releasably retained on the body 12 by suitable means, and when removed provides access to the chamber 14 in order to allow the cartridge 20 or ampoule 22 to be inserted and removed as necessary. The dispensing tip 18, in particular when incorporating the filter 40, may be provided as a consumable item to be replaced along with the cartridge 20. Thus the ampoule 22, dispensing tip 18 and filter 40 may be provided as a combined unit, in the form of the cartridge 20 as illustrated in Figure 13, whereby the filter 40 is captured within the open end 26 of the outer sleeve 24 which open end 26 may then be captured within the dispensing tip 18.

The liquid dispenser 10 of the present invention therefore provides a simple, ergonomic and functional means of dispensing liquids in safe, reliable and controlled manner.

## Claims

1. A liquid dispenser comprising a housing defining a chamber for receiving an ampoule of a liquid to be dispensed, the body further defining an outlet to enable the liquid to be dispensed from the chamber; an actuator displaceable relative to the housing; wherein a section of the housing is adapted to permit a least a section of the actuator to be displaced into the chamber to apply a force, in use, the ampoule.

2. A liquid dispenser according to claim 1 in which the section of the housing permitting access to the chamber is defined by a window in the housing.

3. A liquid dispenser according to claim 2 in which the actuator comprises an abutment shaped and dimension to pass through the window.

4. A liquid dispenser according to claim 2 or 3 in which the actuator comprises a cantilever arm projecting from the housing.

5. A liquid dispenser according to claim 4 in which the abutment is located at or adjacent to a free end of the cantilever arm.

6. A liquid dispenser according to any preceding claim in which the actuator is reversibly displaceable relative to the housing.

7. A liquid dispenser according to any preceding claim in which the actuator is hingedly mounted to the housing.

8. A liquid dispenser according to any of claims 4 to 7 in which the actuator is biased away from the housing.

9. A liquid dispenser according to any of claims 4 to 8 in which at least a portion of the actuator is formed from a resiliently deformable material.

10. A liquid dispenser according to any preceding claim in which the outlet comprises a nozzle in fluid communication with the chamber.

11. A liquid dispenser according to claim 9 in which the nozzle is releasably retained on the housing in order to permit access to the chamber.

12. A liquid dispenser according to any preceding claim in which the housing comprises a substantially cylindrical hollow body.

13. A liquid dispenser according to any preceding claim in which the housing is rigid.

14. A liquid dispenser according to any preceding claim comprising an ampoule of a liquid shaped and dimensioned to be received in the chamber.

15. A liquid dispenser according to claim 14 in which the ampoule comprises a breachable wall.
